# EUROPEAN PATENT APPLICATION

(11) **EP 0 538 945 A1**
(43) Date of publication of application: **28.04.1993**
(21) Application number: 92203188.5
(22) Date of filing: 19.10.1992
(51) Int. Cl.: C07D 243/24, C07D 403/12, A61K 31/55

(54) **Benzodiazepine derivatives, and their use as antagonists of gastrin and/or cholecystokinin**

(30) Priority: 24.10.1991 GB 9122591; 24.10.1991 GB 9122551; 24.10.1991 GB 9122540
(71) Applicant: GLAXO GROUP LIMITED, Greenford, Middlesex UB6 0NN (GB)
(72) Inventor: Carr, Robin Arthur Ellis, Glaxo Group Research Ltd, Ware, Hertfordshire SG12 0DP (GB); Pass, Martin, Glaxo Group Research Ltd., Ware, Hertfordshire SG12 0DP (GB); Shah, Pritom, Glaxo Group Research Ltd., Ware, Hertfordshire SG12 0DP (GB)
(74) Representative: Filler, Wendy Anne, Dr.

(57) **Abstract**

(I) Compounds ofgeneral formula (I) 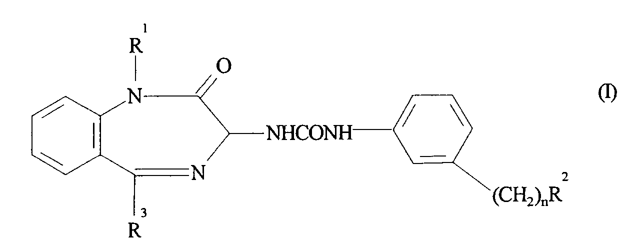
wherein;
R¹ represents a group selected from CH₂CONR⁴R⁵, XYR⁶, phenyl, C₃₋₇-cycloalkyl or C₁₋₆alkyl, optionally substituted by a hydroxy, phenyl, C₁₋₆alkoxycarbonyl, C₃₋₇cycloalkyl or adamantyl group;
R² represents a group selected from NR⁷SO₂CF₃, SO₂NR⁷COR⁸, CONR⁷SO₂R⁸,
or a tetrazole, carboxamidotetrazole, or 3-trifluoromethyl-1,2,4-triazole group in which the tetrazole or triazole moiety may be substituted on one of the nitrogen atoms by a C₁₋₄alkyl group;
R³ is phenyl optionally substituted by one or two halogen atoms;
R⁴ and R⁵ which may be the same or different each independently represent a hydrogen atom, or a phenyl or C₁₋₄alkyl group or NR⁴R⁵ represents a saturated 5- to 7- membered nitrogen containing heterocyclic ring, optionally substituted by 1 or 2 methyl groups;
R⁶ represents a group selected from C₁₋₆alkyl, optionally substituted phenyl, C₃₋₇cycloalkyl or adamantyl;
R⁷ represents hydrogen or a C₁₋₄alkyl group;
R⁸ represents a C₁₋₄alkyl group,
X is a C₁₋₃ straight or branched alkylene chain;
Y represents a group selected from -C=O, C(OR⁹)₂ or C(SR⁹)₂ wherein R⁹ is C₁₋₃alkyl or the two R⁹ groups together form a C₂₋₄alkylene chain;
n is zero or 1; are modulators of gastrin and or CCK.

## Description

This invention relates to novel 1,4-benzodiazepine derivatives, to processes for their preparation, to pharmaceutical compositions containing them and to their use in medicine.

A number of 3 substituted 1,4-benzodiaepine derivatives have been described as antagonists of gastrin and/or choleystokinin (CCK). Such compounds are disclosed in published European patent applications, 167919, 167920, 284256, 434360, 434364 and 434369.

We have now discovered a new class of 3 substituted 1,4-benzodiazepine derivatives which advantageously modulate the effects of gastrin and/or cholecystokinin in mammals.

Thus, the invention provides compounds of general formula (I) 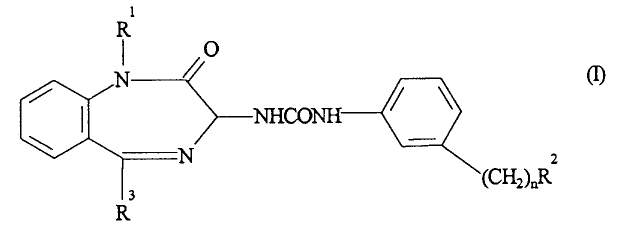
wherein
R¹ represents a group selected from CH₂CONR⁴R⁵, XYR⁶, phenyl , C₃₋₇-cycloalkyl or C₁₋₆alkyl, optionally substituted by a hydroxy, phenyl, C₁₋₆alkoxycarbonyl, C₃₋₇cycloalkyl or adamantyl group;
R² represents a group selected from NR⁷SO₂CF₃, SO₂NR⁷COR⁸, CONR⁷SO₂R⁸, or a tetrazole, carboxamidotetrazole, or 3-trifluoromethyl-1,2,4- triazole group in which the tetrazole or triazole moiety may be substituted on one of the nitrogen atoms by a C₁₋₄alkyl group;
R³ is phenyl optionally substituted by one or two halogen atoms;
R⁴ and R⁵ which may be the same or different each independently represent a hydrogen atom, or a phenyl or C₁₋₄alkyl group or NR⁴R⁵represents a saturated 5- to 7- membered nitrogen containing heterocyclic ring, optionally substituted by 1 or 2 methyl groups,
R⁶ represents a group selected from C₁₋₆alkyl, optionally substituted phenyl, C₃₋₇cycloalkyl or adamantyl;
R⁷ represents hydrogen or a C₁₋₄alkyl group;
R⁸ represents a C₁₋₄alkyl group,
X is a C₁₋₃ straight or branched alkylene chain;
Y represents a group selected from -C=O, C(OR⁹)₂ or C(SR⁹)₂ wherein R⁹ is C₁₋₃alkyl or the two R⁹ groups together form a C₂₋₄alkylene chain;
n is zero or 1; and pharmaceutically acceptable salts and solvates thereof.

It will be appreciated that compounds of formula (I) possess at least one asymmetric carbon atom (namely the carbon atom occupying the 3-position of the diazepine ring) and the compounds of the invention thus include all stereoisomers and mixtures thereof including the racemates.

In the compounds of formula (I) the term alkyl means a straight or branched chain alkyl group. Thus, C₁₋₆alkyl includes methyl, ethyl, n-propyl, isopropyl, n-butyl, isobutyl, tert-butyl, n-pentyl, isopentyl or neopentyl.

For the groups R⁷,R⁸ and R¹° the term C₁₋₄alkyl also includes C₃₋₄cycloalkyl e.g. cyclopropyl and cyclobutyl.

The term C₃₋₇cycloalkyl includes cyclopropyl, cyclobutyl, cyclopentyl, cyclohexyl and cycloheptyl.

Halogen in the definition of compounds of formula (I) may represent a fluoro, chloro, bromo or iodo substituent.

When NR⁴R⁵ represents a saturated 5- to 7-membered heterocyclic ring this may be for example a pyrrolidine, piperidine or hexamethylenimino ring which rings may be substituted by one or two methyl groups, such as 2,5-dimethyl pyrrolidine.

When R² represents a tetrazole group suitable examples include 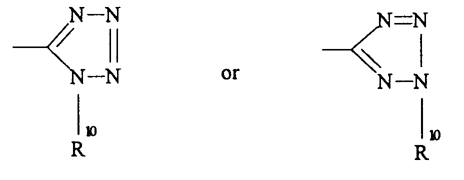
When R² represents a carboxamidotetrazole grouping suitable examples include 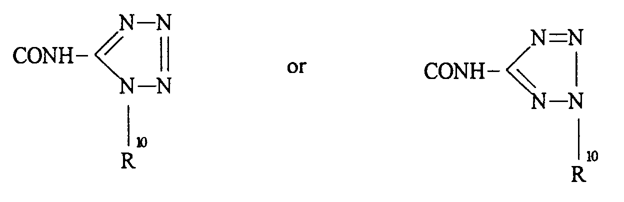
When R² represents a 3-trifluoromethyl 1,2,4-triazole grouping suitable examples include 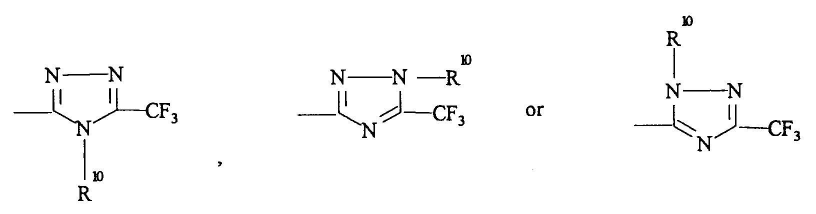
The group R¹⁰ represents hydrogen or a C₁₋₄alkyl group. It will be appreciated that when R¹⁰ represents a hydrogen atom the various isomers for each heterocyclic group are tautomers of that heterocyclic group and all tautomers are included where the formula shows a single tautomer.

When R¹ is the group CH₂CONR⁴R⁵ examples of suitable groups includes those wherein one of R⁴ and R⁵ represents phenyl and the other represents or C₁₋₄alkyl (e.g. methyl or ethyl) or NR⁴R⁵ represents a saturated 5- to 7-membered heterocyclic ring e.g. pyrrolidine.

When R¹ represents the group XYR⁶, examples of suitable groups include those wherein X is methylene, Y is CO or a ketal thereof and R⁶ is C₃₋₄alkyl e.g. isopropyl or t-butyl, C₅₋₇Cycloalkyl e.g. cyclopentyl, phenyl or adamantyl.

When R¹ represents an optionally substituted alkyl group examples of suitable groups include C₄₋₆alkyl e.g. butyl, 3-methylbutyl, 3,3-dimethylbutyl C₂₋₆hydroxyalkyl, alkoxycarbonylalkyl or phenylalkyl.

When R¹ represents the group CH₂CONR⁴R⁵ preferred groups are those wherein one of R⁴ or R⁵ represents phenyl and the other represents ethyl more particularly methyl or NR⁴R⁵ represent a pyrrolidine ring. Most preferably one of R⁴ or R⁵ represents phenyl and the other methyl.

When R¹ represents the group XYR⁶ preferred groups include those wherein X is methylene, Y is CO and R⁶ is C₃₋₄alkyl e.g. t-butyl, cyclopentyl or phenyl.

Other preferred R¹ groups include 3-methyl, butyl and 3,3-dimethylbutyl.

A further preferred class of compounds of formula (I) is that in which R² represents NHSO₂CF₃, SO₂NHCOCH₃, CONHSO₂CH₃ 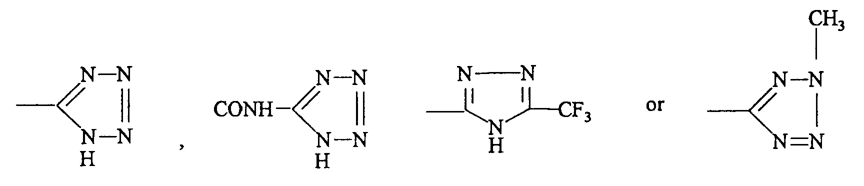
Most preferably R² represents CONHSO₂CH₃ or 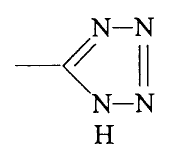
A further preferred class of compounds of formula (I) is that wherein n is zero.

A further preferred class of compounds of formula (I) is that in which R³ represents phenyl or phenyl mono- or di-substituted by fluorine, preferably in the ortho and/or para position(s). Preferably R³ represents unsubstituted phenyl or 2-flurophenyl.

A particularly preferred class of compounds are those wherein R¹ represents the group CH₂CONR⁴R⁵.

A preferred group of compounds according to the invention are those wherein R¹ is a group selected from, 3-methyl butyl, CH₂CONR⁴R⁵ wherein R⁴ represents phenyl and R⁵ represents ethyl or more particularly methyl or NR⁴R⁵ represents pyrrolidine, CH₂COR⁶ wherein R⁶ is t-butyl, cyclopenyl or phenyl;
R² is a group selected from NHSO₂CF₃, SO₂NHCOCH₃, CONHSO₂CH₃, 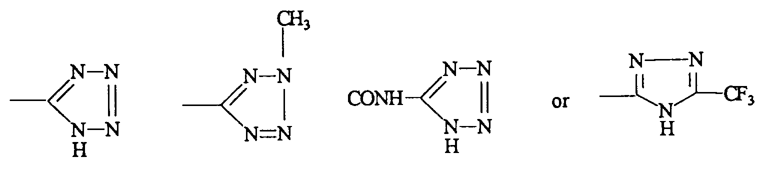
R³ is a phenyl or 2-fluorophenyl group and n is zero or 1. Within this group particularly preferred compounds are those wherein R¹ is CH₂CONR⁴R⁵ and NR⁴R⁵ is pyrrolidine or more especially NCH₃Ph; R² is 5-(1H-tetrazolyl) or more especially CONHSO₂CH₃, n is zero and R³ is phenyl or 2-fluorophenyl.

Preferred compounds according to the invention include those specifically described herein in the examples. Particularly preferred compounds are:
3-[[[[2,3-Dihydro-N-methyl-2-oxo-N,5-diphenyl-1H-1,4-benzodiazepin-3-yl]amino] carbonyl]amino]-N-(methylsulphonyl)benzamide;
2,3-Dihydro-N-methyl-2-oxo-N,5-diphenyl-3-[[[[3-(1H-tetrazol-5-yl)phenyl]amino] carbonyl]amino]-1H-1,4-benzodiazepine-1-acetamide;
5-(2-Fluorophenyl)-2,3-dihydro-N-methyl-2-oxo-N-phenyl-3-[[[[3-(1H-tetrazol-5-yl)phenyl]amino]carbonyl]amino]-1H-1,4-benzodiazepine-1-acetamide;
N-[2,3-Dihydro-2-oxo-1-[2-oxo-2-(1-pyrrolidinyl)ethyl]-5-phenyl-1H-1,4-benzodiazepi n-3-yl]-N'-[3-(1H-tetrazol-5-yl)phenyl]urea;
and specific enantiomers thereof.

The pharmaceutically acceptable salts of the compounds of formula (I) include conventional salts formed for example from pharmaceutically acceptable inorganic or organic acids as well as quaternary ammonium acid addition salts. Examples of suitable salts include hydrochloric, hydrobromic, sulfuric, phosphoric, nitric, perchloric, fumaric, acetic, propionic, succinic, glycolic, formic, lactic, maleic, tartaric, citric, pamoic, malonic, hydroxymaleic, phenylacetic, glutamic, benzoic, salicylic, fumaric, toluenesulphonic, methanesulphonic, naphthalene-2-sulphonic, benzenesulphonic and the like. Other acids such as oxalic, while not in themselves pharmaceutically acceptable, may be useful in the preparation of salts useful as intermediates in obtaining the compounds of the invention and their pharmaceutically acceptable salts.

The compounds of formula (I) in which R⁷ or R¹⁰ are hydrogen may form pharmaceutically acceptable salts with suitable cations. Suitable pharmaceutically acceptable cations include alkali metal (e.g. sodium or potassium) and alkaline earth metal (e.g calcium or magnesium) cations.

References hereinafter to a compound according to the invention includes both compounds of formula (I) and their pharmaceutically acceptable salts and solvates.

The compounds of the invention modulate the effect of gastrin and/or CCK in mammals. In particular compounds of the invention are antagonists of gastrin and/or CCK.

Compounds of the invention have been shown to be antagonists of gastrin as demonstrated by their ability to inhibit pentagastrin-stimulated acid secretion from rat isolated gastric mucosa using the procedure described by J.J. Reeves and R. Stables in Br. J. Pharmac., 1985 86, p.677-684. Compounds of the invention also inhibit pentagastrin-stimulated acid secretion in the conscious Heidenhain pouch dog.

The compounds of the invention have also been shown to be antagonists of CCK, particularly at CCK-B receptors as demonstrated for example by the compound's ability to inhibit the contractile actions of CCK-4 in the presence of a CCK-A antagonist, in the guinea-pig isolated ileum longitudinal muscle-myenteric plexus.

The preparation and use of guinea-pig isolated ileum longitudinal muscle-myenteric plexus has been described by K-H Buchheit et al in Nauyn-Schmeideberg's Arch. Pharmacol, (1985), 329, p36-41 and by V.L. Lucaites et al (1991) in J. Pharmacol. Exp. Ther., 256, 695-703.

The compounds of the invention are therefore useful for the treatment and/or prevention of disorders in mammals, especially humans, where modification of the effects of gastrin or CCK is of therapeutic benefit. Thus the compounds of the invention are useful for the treatment of gastrointestinal disorders especially those where there is an advantage in lowering gastric acidity. Such disorders include peptic ulceration, reflux oesophagitis and Zollinger Ellison syndrome. They may also be useful for the treatment of gastrointestinal disorders such as irritable bowel syndrome, excess pancreatic secretion, acute pancreatitis, motility disorders, antral G cell hyperplasia, fundic mucosal hyperplasia or gastrointestinal neoplasms. The compounds of the invention are also useful for the treatment of central nervous system disorders where CCK and/or gastrin are involved. For example anxiety disorders (including panic disorder, agoraphobia, social phobia, simple phobia, obsessive compulsive disorders, post traumatic stress disorder, and general anxiety disorder), tardive dyskinesia, Parkinson's disease or psychosis. They may also be useful for the treatment of dependency on drugs or substances of abuse and withdrawal, Gilles de la Tourette syndrome, or dysfunction of appetite regulatory systems; as well as the treatment of certain tumours of the lower oesophagus, stomach, intestines and colon. Compounds of the invention are also useful for directly inducing analgesia, or enhancing opiate or non-opiate mediated analgesia, as well as anaesthesia or loss of the sensation of pain.

The invention therefore provides a compound of formula (I) or a pharmaceutically acceptable salt or solvate thereof for use in therapy, in particular in human medicine.

According to another aspect the invention provides the use of a compound of formula (I) or a pharmaceutically acceptable salt or solvate thereof for the manufacture of a medicament for the treatment of conditions where modification of the effects of gastrin and/or CCK is of therapeutic benefit.

According to a further aspect of the invention we provide a method for the treatment of a mammal, including man, in particular in the treatment of conditions where modification of the effects of gastrin and/or CCK is of therapeutic benefit which method comprises administering an effective amount of a compound of formula (I) or a pharmaceutically acceptable salt or solvate thereof to the patient.

It will be appreciated by those skilled in the art that reference herein to treatment extends to prophylaxis as well as the treatment of established diseases or symptoms.

It will further be appreciated that the amount of a compound of the invention required for use in treatment will vary with the nature of the condition being treated and the age and the condition of the patient and will be ultimately at the discretion of the attendant physician or veterinarian. In general however doses employed for adult human treatment will typically be in the range of 20-2000mg per day e.g 100-500mg per day.

The desired dose may conveniently be presented in a single dose or as divided doses administered at appropriate intervals, for example as two, three, four or more sub-doses per day.

Because compounds of the invention antagonise the function of CCK in animals, they may also be used as feed additives to increase the food intake in animals in daily dosages of around 1mg/kg to 10mg/kg.

While it is possible that, for use in therapy, a compound of the invention may be administered as the raw chemical it is preferable to present the active ingredient as a pharmaceutical formulation.

The invention thus further provides a pharmaceutical formulation comprising a compound of formula (I) or a pharmaceutically acceptable salt thereof together with one or more pharmaceutically acceptable carriers therefor and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, or rectal administration. Oral administration is preferred.

Tablets and capsules for oral administration may contain conventional excipients such as binding agents, for example, syrup, accacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate, or wetting agents such as sodium lauryl sulphate. The tablets may be coated according to methods well known in the art. Oral liquid preparations may be in the form of, for example, aqueous or oily suspensions, solutions emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or ascorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

The composition according to the invention may also be formulated as a depot preparation. Such long acting formulations may be administered by implantation (for example subcutaneously or intramuscularly) or by intramuscular injection. Thus for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

The compositions according to the invention may contain between 0.1 - 99% of the active ingredient, conveniently from 30-95% for tablets and capsules and 3-50% for liquid preparations.

Compounds of general formula (I) and salts thereof may be prepared by the general methods outlined hereinafter. In the following description, the groups R¹-R¹⁰ are as defined for the compounds of formula (I) unless otherwise stated.

According to a first general process (A) compounds of formula (I) may be prepared by reacting an imidazolide of formula (II) 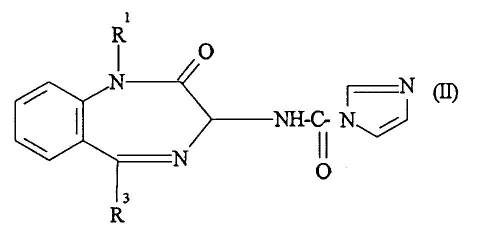
with an aniline of formula (III) or an acid addition salt thereof (in which R² is a defined formula (I) or a group convertible thereto). 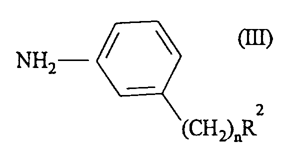
optionally in the presence of a base such as a tertiary amine (e.g. triethylamine). The reaction conveniently takes place in a suitable solvent such as a halogenated hydrocarbon (e.g. dichloromethane), an ether (e.g. tetrahydrofuran) or an amide (e.g. dimethylformamide) or mixtures thereof at a temperature ranging from room temperature to the reflux temperature of the solvent.

In a particular aspect of the process (A) the imidazolide may be formed in situ in which case the aniline of formula (III) will be reacted with a compound of formula (IV) 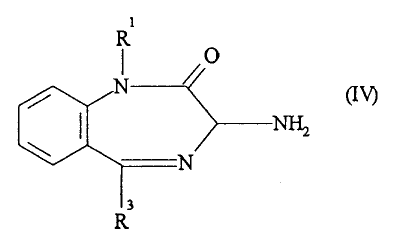
in the presence of carbonyldiimidazole under the aforementioned conditions.

According to a further general process (B) compounds of formula (I) may be prepared by reacting a compound of formula (IV) with an isocyanate of formula (V) 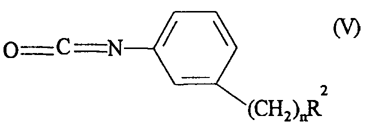
or a carbamoyl chloride of formula (VI) 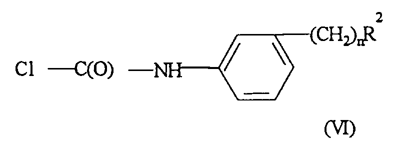
The reaction conveniently takes place in the presence of a suitable solvent such as a halohydrocarbon (e.g. dichloromethane), an ether (e.g tetrahydrofuran) or a nitrile (e.g. acetonitrile) or a mixture thereof at a temperature in the range of 0°C to 80⁰C.

Compounds of formula (II) may be prepared by reacting a compound of formula (IV) with carbonyldiimidazole in the presence of a suitable solvent such as a halogenated hydrocarbon (e.g. dichloromethane) or an ether (e.g. tetrahydrofuran) at a temperature ranging from 0⁰C to 80⁰C, conventionally at room temperature.

Compounds of formula (IV) are either known or may be prepared according to the methods used for the preparation of known compounds. Thus compounds of formula (IV) may be prepared by reduction of the oximes of formula (VII) wherein R¹ is as defined in formula (I) or a group convertible thereto. 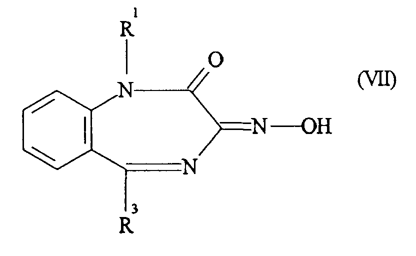
Reduction may be effected for example using catalytic hydrogenation with a suitable metal catalyst such as ruthenium on carbon. The reaction conveniently takes place in a suitable solvent such as an alcohol (e.g. methanol) at a temperatures ranging from 20 to 100°C (e.g. 80°C) under pressure e.g. 80psi.

The oximes of formula (VII) may be prepared by reacting a compound of formula (VIII). 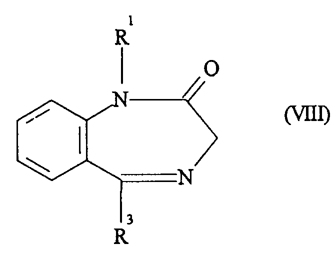
with a strong base such as potassium tert-butoxide followed by a suitable oximating agent such as isoamylnitrite. The reaction conveniently takes place in a suitable solvent such as an ether (e.g. tetrahydrofuran) at a temperature ranging from -30 to 0°C.

Compounds of formula (VIII) may be prepared by reacting a compound of formula (IX) 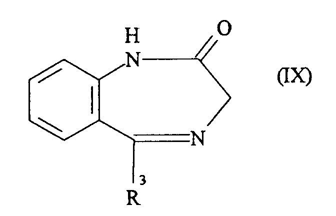
with a suitable alkylating agent such as a compound of formula (X)

hal ― R¹ (X)

where hal represents a halogen (e.g. chlorine or bromine) atom under strongly basic conditions.

Thus the reaction may conveniently be carried out by pretreating the compound of formula (IX) with a strong base such as sodium hydride in a suitable solvent such as an amide (e.g. N, N- dimethylformamide) at a temperature ranging from 0°C to room temperature.

In general, the anilines of formula (III) will be known or may be prepared according to methods used for the preparation of known compounds, for example they may be prepared by reduction of the corresponding nitro compounds. Reduction may be effected for example by catalytic hydrogenation using a suitable metal catalyst such as palladium on carbon in a suitable solvent such as an alcohol (e.g. ethanol) at room temperature.

According to a further process (C) a compound of formula (I) in which R² is as defined in formula (I) or a group convertible thereto may be converted into another compound of formula (I) in which R² is as defined above.

Thus a compound of formula (I) in which R² is carboxamido tetrazole group may be prepared from the corresponding compound wherein R² is a carboxyl group by reaction with 5-aminotetrazole in the presence of a carboxyl activating agent such as carbonyl diimidazole. The reaction may be carried out in a solvent such as acetronitrile.

Compounds of formula (I) where Y represents the group C=O, may be prepared from the corresponding ketals [Y = C(OR⁹)₂] or thioketals [Y = C(SR⁹)₂] by conventional means. Thus for example the ketals may be hydrolysed to the required ketone by treatment with a dilute mineral acid such as hydrochloric acid or an organic acid such as trifluoroacetic, or p-toluenesulphonic acid. The reaction is preferably carried out in a suitable solvent such as dioxan or THF. The thioketals may be hydrolysed by treatment with a silver salt such as AgClO₄ or AgNO₂/Ag₂O or mercuric salt e.g. mercuric chloride in the presence of cadmium or calcium carbonate. The reaction is preferably carried out in an organic solvent such as acetontrile or acetone and water.

Compounds of formula (I) wherein Y is C(OR⁹)₂ may be prepared from the corresponding ketone (Y is C=O) by reaction with the appropriate alcohol R⁹OH or diol in the presence of an acid catalyst such as hydrogen chloride or p-toluene sulphonic acid in an anhydrous solvent such as a hydrocarbon, e.g. benzene or toluene or an ether such as tetrahydrofuran or dioxan.

Compounds of formula (I) wherein Y is C(SR⁹)₂ may be prepared from the corresponding ketone (Y is C=O) by reaction with the appropriate thiol (R⁹SH) or dithiol in the presence of a suitable catalyst such as concentrated hydrochloric acid, trimethylsilyl chloride or boron trifluoride etherate.

Compounds of formula (I) may also be prepared by alkylation of the corresponding compounds of formula (I) wherein R¹ presents hydrogen. The alkylation rection may be carried out using the conditions described above for preparing compounds of formula (VIII) from compounds of formula (IX).

Compounds of formula (I) contain at least one asymmetric carbon atom, namely the carbon atom of the diazepine ring to which the substituted urea grouping is attached. Specific enantiomers of the compounds of formula (I) may be obtained by resolution of the racemic compound using conventional procedures such as salt formation with a suitable optically active acid or by the use of chiral H.P.L.C. Alternatively the required enantiomer may be prepared by the corresponding enantiomeric amine of formula (IV) using any of the processes described above for preparing compounds of formula (I) from the amine (IV). The enantiomers of the amine (IV) may be prepared from the racemic amine (IV) using conventional procedures such as salt formation with a suitably optically active acid.

The following examples, which are non-limiting, illustrate the invention. Temperatures are in °C. "Dried" refers to drying with anhydrous MgSO₄. All chromatography was carried out on silica gel. The following abbreviations are used. T.1.c. - thin layer chromatography; CDI-carbonyldiimidazole; TEA - triethylamine; THF - tetrahydrofuran; HCl - hydrochloric acid; EA - ethyl acetate; DE - diethyl ether; EtOH - ethanol; MeOH - methanol; DMF - N,N- dimethylformamide; DMSO - dimethyl sulfoxide (deuterated for N.m.r's); CDCl₃- deuterochloroform; DCM - dichloromethane; KO^{t}Bu - potassium tert-butoxide; CHCl₃- chloroform; ACOH - acetic acid.

### Intermediate 1

### 2,3-Dihydro-N-methyl-2-oxo-N,5-diphenyl-1H-1,4-benzodiazepine-1-acetamide

Sodium hydride (80% in oil; 375mg) was added portionwise over 15min to a solution of 1,3-dihydro-5-phenyl-2H-1,4-benzodiazepin-2-one (2.36g) in dry DMF (25ml) at 0⁰ under nitrogen. After 30min the resulting slurry was treated with a solution of 2-bromo-N-methyl-N-phenylacetamide (2.7g) in DMF (5ml) and stirring was continued at 0⁰ for 1h 20min and at 23⁰ for 1.5h. The yellow solution was then cautiously treated with water and extracted with EA. The combined extracts were washed with saturated brine, dried and evaporated. The residual yellow oil was chromatographed with hexane-EA (1:1) as eluent to give the title compound (3.58g) as a cream foam, m.p. 68-75⁰ dec.
T.l.c. hexane-EA (1:1), Rf 0.11

### Intermediate 2

### 2,3-Dihydro-3-(hydroxyimino)-N-methyl-2-oxo-N,5-diphenyl-1H-1,4-benzodiazepine-1-acetamide

KO^{t}Bu (1.14g) was added portionwise over 5min to a solution of Intermediate 1 (3.55g) in dry THF (180ml) at -20⁰ under nitrogen. After 30min at -20⁰, the solution was treated with isoamyl nitrite (1.6ml) and stirring was continued at -25⁰ to -10⁰ for 2h whereupon phosphate buffer (pH6; 200ml) was added carefully to quench the reaction. The mixture was extracted with EA and the combined extracts then washed with saturated brine, dried and evaporated. The residual foam was triturated with DE and the solid filtered off to give the title compound (2.3g) as a yellow powder, m.p. 152-5⁰dec.
T.l.c.EA-hexane (4:1), Rf 0.42

### Intermediate 3

### 3-Amino-2,3-dihydro-N-methyl-2-oxo-N,5-diphenyl-1H-1,4-benzodiazepine-1-acetamide

A mixture of Intermediate 2 (3.19g), 5% ruthenium on carbon (950mg) and MeOH (200ml) was hydrogenated at 80⁰ and 80psi for 17h then cooled and filtered. The filtrate was evaporated and the residue chromatographed with 2→4% MeOH in DCM as eluent to give the title compound (907mg) as an off-white foam, m.p. 110⁰ dec.
T.l.c.DCM-MeOH (9:1), Rf 0.49

### Intermediate 4

### N-[2,3-Dihydro-2-oxo-1-[2-oxo-2-(1-pyrrolidinyl)ethyl]-5-phenyl-1H-1,4-benzodiazepi n-3-yl]-1H-imidazole-1-carboxamide

CDI (178mg) was added to a solution of 3-amino-2,3-dihydro-1-[2-oxo-2-(1-pyrrolidinyl)ethyl]-5-phenyl-2H-1,4-benzodiazepin-2-one (400mg) in dry DCM (3ml) at 23⁰ under nitrogen. After 1.5h DE (5ml) was added to the solution to give a gum. The mixture was evaporated to dryness and the residue triturated with DE-hexane to give the title compound (374mg) as a white solid with m.p. 133⁰ dec.
T.l.c. DCM-MeOH (95:5), Rf 0.17.

### Intermediate 5

### N-(Methylsulphonyl)-3-nitrobenzamide

A solution of CDI (4.1g) in THF (50ml) was treated with a solution of 3-nitrobenzoic acid (4.2g) in dry THF (50ml). The mixture was stirred at 70⁰ for 30min, cooled to room temperature and treated with methane sulphonamide (2.9g). The mixture was stirred for 10min and treated with a solution of diazabicyclo-undecene (3.75ml) in dry THF (25ml). The resulting mixture was stirred at room temperature for 20h, the solvent was removed by evaporation and the residue partitioned between 2N HCl and EA. The aqueous phase was extracted with EA and the combined organic extracts were dried and evaporated. The residue was triturated with DE and filtered to give the title compound (3.14g) as a colourless solid.
T.l.c.hexane-EA-acetic acid (50:49:1), Rf 0.32.

### Intermediate 6

### 3-Amino-N-(methylsulphonyl)benzamide

A solution of N-(methylsulphonyl)-3-nitrobenzamide (1.5g) in EA (50ml) was hydrogenated at 1atm and room temperature over 5% palladium on carbon (0.15g) for 2½h. The catalyst was removed by filtration and washed with EtOH. The filtrate was evaporated to give the title compound (1.21g) as a colourless solid.
T.l.c.hexane-EA-acetic acid (50:49:1), Rf 0.26

### Intermediate 7

### 1,1,1-Trifluoro-N-[(3-nitrophenyl)methyl]methanesulphonamide

A solution of 3-nitrobenzylamine hydrochloride (5.0g) in dry DCM (50ml) at -30⁰ was treated with TEA (7.3ml) and then with trifluoromethane sulphonic anhydride (4.25ml). The reaction was warmed to room temperature and stirred for 18h. The mixture was poured into 1N HCl and the aqueous phase extracted with DCM. The combined organic extracts were dried and evaporated. The residue was chromatographed on silica, eluting with 4:1 hexane-EA to give the title compound as a yellow solid (4.40g).
T.l.c.hexane-EA (4:1), Rf 0.53

### Intermediate 8

### N-[(3-Aminophenyl)methyl]-1,1,1-trifluoromethanesulphonamide

A solution of 1,1,1-trifluoro-N-[(3-nitrophenyl)methyl]methanesulphonamide (2.0g) in EtOH (30ml) was hydrogenated at 1atm and room temperature over 5% palladium on carbon (0.20g) for 18h. The catalyst was removed by filtration and washed with EtOH. The filtrate was evaporated to give the title compound (1.20g) as a pale brown solid.
T.l.c. hexane-EA (2:1), Rf 0.41

### Intermediate 9

### 1,1-Dimethylethyl [3-(1H-tetrazol-5-yl)phenyl]carbamate

TEA (614mg) was added to a stirred solution of 3-(1H-tetrazol-5-yl)benzenamine (490mg) in DCM-MeOH (1:1;15ml). Di-tert-butyl dicarbonate (1.3g) was added and the reaction allowed to stir overnight. The solvent was removed and the residue partitioned between 1N sodium hydroxide solution and ethyl acetate. The aqueous layer was separated, acidified with dilute citric acid and extracted with ethyl acetate. The combined ethyl acetate extracts were washed with water, saturated brine, dried and evaporated to give the title compound as a white solid (511mg).
T.l.c.DE Rf 0.5 (Streaks).

### Intermediate 10

### 1,1-Dimethylethyl [3-(2H-2-methyltetrazol-5-yl)phenyl]carbamate

A mixture of the Intermediate 9 (475mg), potassium carbonate (500mg) and methyl iodide (0.12ml) in DMF (5ml) was heated at 60°C for 16h. The reaction mixture was poured into water and extracted with ethyl acetate. The combined organic extracts were washed with water, saturated brine, dried and evaporated to give a white solid. This was chromatographed on silica (eluting with DCM) to give the title compound (322mg).
T.l.c. DE Rf 0.7

### Intermediate 11

### 3-(2H-2-Methyltetrazol-5-yl)benzenamine

Ethereal HCl (1M; 4ml,) was added to a solution of Intermediate 10 (309mg), in dichloromethane (5ml) at 0°C. The reaction mixture was allowed to stir under nitrogen for 24h. The volatiles were removed in vacuo and the residue suspended in DCM (5ml). Trifluoroacetic acid (5ml) was added and the clear solution allowed to stand at room temperature under nitrogen for 3h. The solvent was evaporated and the residue dried in vacuo to give the title compound as a white solid (330mg).
T.l.c. DE Rf 0.56

### Intermediate 12

### 3-[[[[2,3-Dihydro-2-oxo-1-[2-oxo-2-(1-pyrrolidinyl)ethyl]-5-phenyl-1H-1,4-benzodiazepin-3-yl]amino]carbonyl]amino]benzoic acid

A mixture of intermediate 4 (493mg) and 3-aminobenzoic acid (200mg) in dry DCM (4ml) was treated with triethylamine (0.2ml) and the reaction mixture allowed to stir at room temperature overnight. The reaction mixture was diluted with dichloromethane and washed with dilute HCl. The organic layer was dried (Na₂SO₄) and concentrated to give a white foam. This was triturated with DCM/ether and filtered off to give the title compound as a white powder (136mg), m.p. 185-188°.
T.l.c. DCM-MeOH (9:1), Rf 0.48

### Intermediate 13

### N-[(3-Nitrophenyl)sulphonyl]acetamide

A mixture of 3-nitrobenzenesulphonamide (5.0g) and acetylchloride (4.4ml) in acetic acid (4ml) was heated at 50° for 30mins. The solvent was removed by evaporation and the residue partitioned between ethyl acetate and water. The organic portion was dried (Na₂SO₄) and evaporated then the residue was chromatographed, eluting with hexane-ethyl acetate (2:1) to give the title compound (2.8g) as a white solid, m.p. 185-186°
T.l.c. Hexane-EA (1:1) R.f. 0.39

### Intermediate 14

### N-[(3-Aminophenyl)sulphonyl]acetamide

A solution of intermediate 13 (1.0g) in absolute ethanol (40ml) was hydrogenated at 1atm and room temperature over 5% palladium on carbon (0.10g) for 64 hrs. The catalyst was removed by filtration through hy-flo and the filtrate was evaporated to give the title compound (0.623g) as a white solid.
T.l.c. MeOH-acetic acid-DCM (5:1:94) R.f 0.32

### Intermediate 15

### 5-[(3-Nitrophenyl)methyl]tetrazole

A mixture of (3-nitrophenyl) acetonitrile (3.0g), sodium azide (1.44g) and ammonium chloride (1.88g) in DMF (10ml) was heated at 100° for 24h. The reaction mixture was partitioned between EA and dilute sodium hydroxide solution. The sodium hydroxide layer was separated and extracted once more with EA. These EA extracts were discarded. The sodium hydroxide layer was acidified with conc. HCl and extracted with EA. The combined EA extracts were washed with water, saturated brine, dried and evaporated to give a white solid. This was triturated with DE and filtered to give the title compound as a white powder (2.9g).
T.l.c. DE, Rf 0.35

### Intermediate 16

### 3-(1H-Tetrazol-5-ylmethyl)benzenamine hydrochloride

5-[(3-Nitrophenyl)methyl]tetrazole (1g) was hydrogenated at room temperature and pressure over 5% palladium-on-carbon (200mg) in ethanol (25ml) containing conc. HCl (0.1ml). After 3h the catalyst was removed by filtration and the filtrate concentrated to give the title compound as a beige gum (825mg).
T.l.c. DCM-MeOH (9:1), Rf 0.37
δ (DMSO) 4.25 (2H,s); 6.85-6.98 (3H,m); 7.25 (1H,t)

### Intermediate 17

### 3-Nitrobenzoic acid 2-(2,2,2-trifluoro-1-iminoethyl)hydrazide

Trifluoroacetamidine (2.74g) was treated with ethereal HCl (1M;25ml) and the mixture allowed to stir at room temperature for 30 min. The volatiles were removed in vacuo to give trifluoroacetamidine hydrochloride. EtOH(50ml) was added followed by 3-nitrobenzoic acid hydrazide (4.43g,) and the mixture stirred at room temperature for 24h. The EtOH was evaporated off in vacuo and the residue washed with water and EA to give a white solid at the interface. This was filtered off and dried in vacuo to give the title compound (2.2g)
T.l.c. EA-Hexane (1:1) Rf 0.4

### Intermediate 18

### 3-(3-Nitrophenyl)-5-(trifluoromethyl)-1H-1,2,4-triazole

Intermediate 17 (1.5g) was heated at reflux with 3N sodium hydroxide solution (30ml) for 1 h. The reaction mixture was acidified with dilute HCl and extracted with ethyl acetate. The combined organic extracts were washed with water, saturated brine, dried and concentrated to give a brown gum. This was chromatographed (eluting with EA-Hexane; 3:7) to give the title compound as a pale yellow solid (1.25g)
T.l.c. EA-Hexane (1:1) Rf 0.63

### Intermediate 19

### 3-[5-(Trifluoromethyl)-1H-1,2,4-triazol-3-yl]benzenamine hydrochloride

Intermediate 18 (540mg) was hydrogenated at room temperature and pressure over 10% palladium on carbon (100mg) in ethanol (25ml) containing conc. HCl (0.1ml), for 24h. The catalyst was removed by filtration and the filtrate concentrated in vacuo to give the title compound as a white solid (560mg).
T.l.c. EA-Hexane (1:1) Rf 0.33

### Intermediate 20

### 5-(2-Fluorophenyl)-2,3-dihydro-N-methyl-2-oxo-N-phenyl-1H-1,4-benzodiazepine-1-acetamide

Sodium hydride (80% dispersion in oil; 0.29g) was added portionwise to a stirred solution of 5-(2-fluorophenyl)-1,3-dihydro-2H-1,4- benzodiazepin-2-one (2.0g) in DMF (70ml) at 0° under nitrogen. After 0.75h 2-bromo-N-methyl-N-phenylacetamide (2.1g) in DMF (20ml) was added and stirring was continued at 0° for 3h. The mixture was then poured into water and extracted with EA. The combined organic washings were washed with water, dried and evaporated. The residue was purified by flash chromatography eluting with DCM-MeOH (98:2) to give the title compound as a white solid (2.5g), m.p. 230° dec.
T.l.c.DCM-MeOH (95:5), Rf 0.32

### Intermediate 21

### 5-(2-Fluorophenyl)-,2,3-dihydro-3-(hydroxyimino)-N-methyl-2-oxo-N-phenyl-1H-1,4-benzodiazepine-1-acetamide

Potassium-t-butoxide (0.8g) was added portionwise to a stirred solution of Intermediate 20 (2.5g) in THF (100ml) cooled to -55° under nitrogen. After 0.75h isoamyl nitrite (1.0g) was added dropwise. Stirring was continued at -40° to -30° for 2h then the mixture was poured into pH6.5 phosphate buffer (300ml) and extracted into EA. The combined organic extracts were washed with saturated brine, dried and evaporated. The residue was purified by flash chromatography, eluting with EA-hexane (50:50) to give the title compound as a yellow foam (2.3g), m.p. 138-141°.
T.l.c.DCM-MeOH (95:5), Rf 0.30

### Intermediate 22

### 3-Amino-5-(2-fluorophenyl)-2,3-dihydro-N-methyl-2-oxo-N-phenyl-1H-1,4-benzodiazepine-1-acetamide

A solution of Intermediate 21 (2.2g) in EtOH (35ml) and water (8ml) was hydrogenated at 60°, 70p.s.i. using 5% ruthenium on carbon (0.48g) as a catalyst. After 12h the reaction mixture was filtered through celite and evaporated. The residue was purified by flash chromatography, using MeOH (2% to 4%) in DCM as eluent to give the title compound as a white foam (1.55g), m.p. 115-120°.
T.l.c.DCM-MeOH (95:5), Rf 0.18

### Intermediate 23

### 3-Amino-5-(2-fluorophenyl)-2,3-dihydro-N-methyl-2-oxo-N-phenyl-1H-1,4-benzodiazepine-1-acetamide (isomer 1)

The Intermediate 22 amine (550mg) was resolved by chiral HPLC:

| | |
|---|---|
| Column: | CHI-D-PGC 5µ 25cm x 20mm id |
| Eluent: | hexane-CHCl₃-MeOH (60:40:3) |
| Flow rate: | 20ml min⁻¹ |
| Detection: | u.v. @ 254nm |
| Loading: | 10mg/ml/injection |

to give Isomer 1 (227mg), 96% enantiomerically pure by peak heights, as a white solid, m.p. 165° dec.
T.l.c. DCM-MeOH (9:1), Rf 0.4

### Intermediate 24

### 3-Amino-5-(2-fluorophenyl)-2,3-dihydro-N-methyl-2-oxo-N-phenyl-1H-1,4-benzodiazepine-1-acetamide (isomer 2)

The intermediate 22 amine (550mg) was resolved by chiral HPLC:

| | |
|---|---|
| Column: | CHI-D-PGC 5µ 25cm x 20mm id |
| Eluent: | hexane-CHCl₃-MeOH (60:40:3) |
| Flow rate: | 20ml min⁻¹ |
| Detection: | u.v. @ 254nm |
| Loading: | 10mg/ml/injection |

to give Isomer 2 (223mg), 96% enantiomerically pure by peak heights, as a white solid, m.p. 131° dec.
T.l.c. DCM-MeOH (9:1), Rf 0.4

### Intermediate 25

### 2-Bromo-N-[2-(2-fluorobenzoyl)phenyl]acetamide

Bromoacetylbromide (24.6g) in DCM (35ml) was added dropwise to a stirred solution of 2-amino-2'-fluoro-benzophenone in DCM (150ml) and water (10ml) at 0° under nitrogen. After 2h water (125ml) was added and the organic phase was separated, washed with water, dried and evaporated to give a residue which was triturated with hexane to give the title compound as a cream solid (38.0g), m.p. 110-112°.
T.l.c. (alumina) DCM, Rf 0.69

### Intermediate 26

### 5-(2-Fluorophenyl)-1,3-dihydro-2H-1,4-benzodiazepin-2-one

A suspension of Intermediate 25 (38g) in MeOH (700ml) at 0°C was saturated with ammonia gas and then was allowed to warm to 23°. After stirring for 18h the yellow solution was purged with nitrogen then evaporated. The residue was dissolved in DCM (500ml) and was washed with saturated brine. The aqueous layer was re-extracted with DCM and the combined organic washings were dried and evaporated. The resultant solid was triturated with EA and filtered to give the title compound as a white solid (9.9g), m.p. 175-177°.
T.l.c. EA-hexane (50:50), Rf 0.13

### Intermediate 27

### 1-[[5-(2-Fluorophenyl)-2,3-dihydro-2-oxo-1H-1,4-benzodiazepin-1-yl]acetyl]pyrrolidine

Sodium hydride (80% dispersion in oil; 1.2g) was added portionwise to a stirred solution of Intermediate 26 (8.0g) in DMF (180ml) at 0° under nitrogen. After ½h N-bromoacetylpyrrolidine (7.3g) in DMF (20ml) was added and stirring was continued at 0° for 3h. The mixture was poured into water (500ml) and extracted twice with EA. The combined organic washings were dried and evaporated to give a residue which was triturated with DE to give the title compound as a white solid (6.4g), m.p. 172-174°.
T.l.c. MeOH-DCM (5:95), Rf 0.24

### Intermediate 28

### 1-[[5-(2-Fluorophenyl)-2,3-dihydro-3-(hydroxyimino)-2-oxo-1H-1,4-benzodiazepin-1-yl ]acetyl]pyrrolidine

Potassium-t-butoxide (2.1g) was added portionwise to a stirred solution of Intermediate 27 (6.1g) in THF (250ml) at -40° under nitrogen. After 0.75h isoamyl nitrite (2.6g) was added dropwise. Stirring was continued at -30° for 1.5h then the mixture was poured into pH6.5 phosphate buffer (500ml) and extracted into EA. The combined organic extracts were washed with saturated brine, dried and evaporated to give a residue which was triturated with DE to give the title compound as a pale yellow solid (5.9g), m.p. 158° dec.
T.l.c. DCM-MeOH (95:5), Rf 0.17

### Intermediate 29

### 1-[[3-Amino-5-(2-fluorophenyl)-2,3,dihydro-2-oxo-1H-1,4-benzodiazepin-1-yl]acetyl] pyrrolidine

A slurry of Intermediate 28 (5.9g) in EtOH (100ml) and water (20ml) was hydrogenated at 60°, 70p.s.i. using 5% ruthenium on carbon (1.4g) as a catalyst. After 18h the reaction mixture was filtered through celite and evaporated. The residue was purified by flash chromatography using MeOH (1 to 5%) in DCM as eluent to give the title compound as a white solid (4.4g), m.p. 86-88°.
T.l.c. DCM-MeOH (95:5), Rf 0.21

### Intermediate 30

### N-[2,3-Dihydro-N-methyl-2-oxo-N,5-diphenyl-1H-1,4-benzodiazepin-3-yl]-1H-imidazole-1-carobxamide

A solution of CDI (1.3g) in dry DCM (10ml) was added to a stirring solution of Intermediate 3 (3.0g) in dry DCM (20ml) under nitrogen. After 90min the solvent was removed by evaporation to give the title compound (4.3g) as a buff solid foam.
T.l.c. MeOH-DCM (5:95) Rf 0.37
IR 661, 699, 743, 1010, 1061, 1382cm⁻¹

### Intermediate 31

### 3-[[[[2,3-Dihydro-N-methyl-2-oxo-N,5-diphenyl-1H-1,4-benzodiazepin-3-yl]amino]carbonyl]amino]benzoic acid

A mixture of Intermediate 30 (1.02g) and 3-aminobenzoic acid (425mg) in dry THF (25ml) was heated under N₂ at 70° for 96hrs. The solvent was removed by evaporation and the residue dissolved in DCM (100ml). This was washed with 2N HCl (3x40ml) dried and evaporated. The residue was columned (MeOH-AcOH-DCM 3:1:96) to give the title compound (6.45mg) as a white solid.
T.l.c. (5%MeOH-DCM) Rf = 0.23
IR 1220, 1279, 1378, 1595cm⁻¹

### Intermediate 32

### 2,3-Dihydro-N-ethyl-2-oxo-N,5-diphenyl-1H-1,4-benzodiazepine-1-acetamide

NaH (80% in oil; 155mg) was added to a solution of 1,3-dihydro-5-phenyl-2H-1,4-benzodiazepin-2-one (975mg) in dry DMF (10ml) at 0°under nitrogen. After 40 min a solution of 2-bromo-N-ethyl-N-phenylacetamide (1.2g) in dry DMF (2ml) was added and the mixture was stirred for 2.25h as the ice-bath was allowed to warm. The resulting solution was poured cautiously into water (75ml) and extracted with EA (2x50ml). The combined extracts were washed consecutively with water (50ml) and saturated brine (50ml) then dried and evaporated. The residual gum was chromatographed with EA-hexane (2:1) as eluent to give the title compound (1.14g) as a foam m.p. 95° dec
T.l.c. (1:1 hexane-EA) Rf 0.1

### Intermediate 33

### 2,3-Dihydro-3-(hydroxyimino)-N-ethyl-2-oxo-N,5-diphenyl-1H-1,4-benzodiazepine-1-acetamide

KO^{t}Bu (343mg) was added to a solution of Intermediate 32 (1.107g) in dry THF (50ml) at -20° under nitrogen. After 40min at <⁻15° the solution was treated with isoamyl nitrite (420mg) and after a further 40 min with more KO^{t}Bu (100mg). The solution was stirred at -15°-10° for 1.5h then poured into phosphate buffer solution (pH6.5;75ml). The organic layer was separated and the aqueous phase re-extracted with EA (50ml). The combined organic phases were washed with saturated brine (50ml), dried and evaporated. The residue was chromatographed with EA-hexane (2:1) as eluent to give the title compound (820mg) as a foam, m.p. 123° foams.
T.l.c. (3:1 EA-hexane) Rf 0.47

### Intermediate 34

### 3-Amino-2,3-dihydro-N-ethyl-2-oxo-N,5-diphenyl-1H-1,4-benzodiazepine-1-acetamide

A Cook hydrogenation vessel was thoroughly dried and flushed with nitrogen then charged with 5% ruthenium on charcoal (200mg) and a solution of Intermediate 33 (779mg) in absolute EtOH (50ml). The mixture was hydrogenated at 80° and 80psi, pressure for 17h, allowed to cool then filtered through hyflo, and the filtrate evaporated. The residue was chromatographed with 3% MeOH in DCM as eluent to give the title compound (344g) as a crunchy off-white foam, m.p. 100° dec.
T.l.c. (95:5 DCM-MeOH) Rf 0.06

### Intermediate 35

### N-[2,3-Dihydro-1-(3-methylbutyl)-2-oxo-5-phenyl-1H-1,4-benzodiazepin-3-yl]-1H-imidazole-1-carboxamide

CDI (30mg) was added to a solution of 3-amino-1,3-dihydro-1-(3-methylbutyl)-5-phenyl-2H-1,4-benzodiazepin-2-one (598mg) in dry THF (5ml) at 23⁰ under nitrogen. After 1h the solution was poured into water and extracted with EA. The combined extracts were washed with saturated brine, dried and evaporated to give the title compound (805mg) as a brown crunchy foam, m.p. 80⁰ dec.
T.l.c. DCM-MeOH (9:1), Rf 0.52
δ (CDCl₃) 0.7-0.9(6H,dd); 1.3-1.5(3H,m); 3.65-3.8(1H,m); 4.35- 4.5(1H,m); 5.45-5.55(1H,d); 7.05-7.75(12H,m); 8.2-8.25(1H,s).

### Intermediate 36

### 1-(3,3-Dimethyl-2-oxobutyl)-1,3-dihydro-5-phenyl-2H-1,4-benzodiazepin-2-one

80% Sodium hydride in oil (368mg) was added portionwise over 10min to a solution of 1,3-dihydro-5-phenyl-2H-1,4-benzodiazepin-2-one (2.36g) in dry DMF (25ml) at 0⁰ under nitrogen. After 50min the cream foam was treated with a solution of 1-bromopinacolone (2.3g) in dry DMF (5ml) and stirring was continued at 0-10⁰ for 1.75h and at 23⁰ for 2h. The clear yellow solution was then poured cautiously into water and extracted with EA. The combined extracts were washed with saturated brine (100ml), dried and evaporated. The residual orange oil was chromatographed with hexane-EA (3:1 to 2:1) as eluent to give a yellow gum. Trituration with DE gave a white solid which was filtered off and dried in vacuo to give the title compound (2.25g) as white crystals, m.p. 134-6⁰.
T.l.c. (2:1 EA-hexane), Rf 0.46
δ (CDCl₃) 1.15-1.35(9H,s); 3.85-3.95 (1H,d); 4.4-4.55(1H,d); 4.75- 4.85(1H,d); 5.0-5.15(1H,d); 7.0-7.7(9H,m).

### Intermediate 37

### 1-(3,3-Dimethyl-2-oxobutyl)-5-phenyl-1H-1,4,-benzodiazepin-2,3-dione 3-oxime

Potassium t-butoxide (814mg) was added over 5min to a solution of Intermediate 36 (2.2g) in dry THF (130ml) at -20⁰ under nitrogen. After 30min, the yellow solution was treated with isoamyl nitrite (1.15ml) and stirring continued at -25⁰ to +10⁰ for 4h. The orange solution was then carefully quenched with phosphate buffer solution (pH6.5; 150ml) and extracted with EA. The combined extracts were washed with saturated brine, dried and evaporated. The residue was chromatographed with hexane-EA (3:1→2:1→ 1:1) as eluent to give the title compound (1.08g) as a pale yellow foam m.p. 110⁰ (dec).
T.l.c. (3:1 hexane-EA), Rf 0.04
δ (CDCl₃) 1.15-1.35(9H,s); 4.85-5.0(2H,br.s); 7.15-7.95 (10H,m)

### Intermediate 38

### 3-Amino-1-(3,3-dimethyl-2-oxobutyl)-1,3-dihydro-5-phenyl-2H-1,4-benzodiazepin-2-one

A mixture of Intermediate 37 (1g) 5% ruthenium on carbon (380mg) and methanol (75ml) was hydrogenated at 80⁰C and 80psi for 20h. The cooled mixture was filtered through hyflo and the filtrate evaporated. The residue was chromatographed with 2 to 3% methanol in DCM as initial eluent. Further elution with 5% methanol in DCM gave the title compound (395mg) as a crunchy cream foam, m.p. 70-72⁰dec.
T.l.c. (95:5 DCM-MeOH), Rf0.2
δ (CDCl₃) 1.15-1.35(9H,s); 1.7-2.7(2H,br.s); 4.5-4.65(2H,m); 5.0- 5.15(1H,d); 7.05-7.7 (9H,m)

### Intermediate 39

### 3-Amino-2,3-dihydro-2-oxo-5-phenyl-1H-1,4-benzodiazepine

48% HBr in acetic acid (15ml) was added to phenylmethyl[2,3-dihydro-2-oxo-5-phenyl-1,4-benzodiazepin-3-yl]carbamate (1.46g) at 23⁰ under nitrogen. Stirring was continued for 1h with evolution of carbon dioxide, and dissolution of the initial suspension, whereupon the clear yellow solution was partitioned between water (100 + 50ml) and EA (75ml). The combined aqueous extracts were re-extracted with EA (50ml) then carefully basified with 2N sodium carbonate solution and extracted with EA (3x100ml). These latter organic extracts were combined, washed with saturated brine (100ml), dried and evaporated. Trituration of the residue with DE and subsequent filtration and drying in vacuo gave the title compound (639mg) as an off white solid, m.p. 191° dec.
T.l.c. (9:1 DCM-MeOH) Rf 0.29

### Intermediate 40

### N-[2,3-Dihydro-2-oxo-5-phenyl-1H-1,4-benzodiazepin-3-yl]-N-[3-(1H-tetrazol-5-yl)phenyl]urea

CDI (129mg) was added to a suspension of Intermediate 39 (200mg) in dry THF (7ml) at 23° under nitrogen. After 40 min the clear solution was treated with 3-(1H-tetrazol-5-yl)benzenamine (204mg) and the mixture heated under reflux for 1h. On cooling a gum and precipitate formed so DE was added and the supernatant suspension decanted from the gum. The solid was filtered from the suspension, returned to the flask with the gum and this mixture was stirred vigorously with MeOH. The resulting precipitate was filtered off, washed with DE and dried in vacuo at 60° to give the title compound (238mg) as a pale yellow solid, m.p. 207° dec
T.l.c. (9:1 DCM-MeOH) Rf 0.25

### Intermediate 41

### 1-(2-Cyclopentyl-2-oxoethyl)1,3-dihydro-5-phenyl-1,4-benzodiazepin-2-one

Sodium hydride (80% in oil; 597mg) was added portionwise over 15min to a solution of 1,3-dihydro-5-phenyl-2H-1,4-benzodiazepin-2-one (3.76g) in dry DMF (90ml) at 0° under nitrogen. After 30min a solution of 1-bromo-2-cyclopentyl-2-oxoethane (5.8g; 52% pure) in dry DMF (10ml) was added and stirring was continued at 0° for 1h and at 23° for 63h. The mixture was cautiously poured into water and extracted with EA. The combined extracts were washed with saturated brine, dried and evaporated. The residual brown oil was chromatographed with hexane-EA (2:1) as eluent to give after trituration with DE the title compound (2.90g) as a solid, m.p. 104°.
T.l.c. hexane-EA (2:1), Rf 0.18

### Intermediate 42

### 1-(2-Cyclopentyl-2-oxoethyl)-1,3-dihydro-5-phenyl-1,4-benzodiazepin-2,3-dione 3-oxime

KO^{t}Bu (2.06g) was added portionwise over 10min to a solution of Intermediate 41 (2.90g) in dry THF (125ml) at -40° under nitrogen. After 30min at -40° the solution was treated with isoamyl nitrite (1.46ml) and stirring was continued with warming slowly to 0° over 2h. Phosphate buffer (pH6; 500ml) was added carefully to quench the reaction. The mixture was extracted with EA and the combined extracts then washed with saturated brine, dried and evaporated. The residual red oil was chromatographed with hexane-EA (1:1) as eluent to give the title compound (1.41g) as a yellow foam, m.p. 94-100°C.
T.l.c. hexane-EA (1:1), Rf 0.22

### Intermediate 43

### 3-Amino-1-(2-cyclopentyl-2-oxoethyl)-1,3-dihydro-5-phenyl-2H-1,4-benzodiazepin-2-one

A solution of Intermediate 42 (800mg) in MeOH (80ml) and water (20ml) was hydrogenated at 70 psi, 80° in a Cook apparatus for 13 hours using 5% ruthenium on carbon (80mg) as a catalyst. The mixture was filtered through hyflo and concentrated in vacuo. EA (200ml) was added and the mixture washed with saturated brine (200ml), dried and evaporated in vacuo. The crude product was purified by flash chromatography eluting with 2% MeOH-DCM to give product as a foam (284mg).
T.l.c. (5% MeOH-DCM), Rf 0.19

### Example 1

### 2,3-Dihydro-N-methyl-2-oxo-N,5-diphenyl-3-[[[[3-(1H-tetrazol-5-yl) phenyl]amino]carbonyl]amino]-1H-1,4-benzodiazepine-1-acetamide

CDI (81mg) was added to a solution of Intermediate 3 (200mg) in dry THF (3ml) at 23⁰ under nitrogen. After 30min, TEA (0.1ml) and 3-(1H-tetrazol-5-yl)benzenamine hydrochloride (142mg) were added sequentially. Heating was then continued at reflux for 20h whereupon the white suspension was poured into 2N HCl and extracted with EA. The combined extracts were washed with 2N HCl and saturated brine then dried and evaporated. The residue was triturated with EA-DE, filtered off and dried in vacuo to give the title compound (237mg) as a beige solid, m.p. 200-5⁰ dec.
T.l.c.DCM-MeOH (9:1), Rf 0.34
δ (DMSO) 3.1-3.25 (3H,br.s); 4.2-4.6 (2H,br.m); 5.25-5.4 (1H,d); 7.1-7.8 (18H,m); 8.15-8.25 (1H,s); 9.3-9.4 (1H,s)

### Example 2

### N-[2,3-Dihydro-2-oxo-1-[2-oxo-2-(1-pyrrolidinyl)ethyl]-5-phenyl-1H-1,4-benzodiazepi n-3-yl]-N'-[3-(1H-tetrazol-5-yl)phenyl]urea

CDI (266mg) was added to a solution of 3-amino-2,3-dihydro-1-[2-oxo-2-(1-pyrrolidinyl)ethyl]-5-phenyl-1H-1,4-benzodiazepin-2-one (594mg) in dry THF (15ml) at 23⁰ under nitrogen. After 30min the clear yellow solution was treated sequentially with TEA (0.296ml) and 3-(1H-tetrazol-5-yl)benzenamine hydrochloride (420mg) and the resulting suspension was heated under reflux for 23h. It was then poured into EA and washed with 0.6N HCl. The organic phase was treated with DE and hexane to precipitate the product and the solid was filtered off. The filtrate was washed with saturated brine, dried and evaporated to give a second batch of product. The original acid wash was further extracted with EA and these combined extracts were washed with saturated brine, dried and evaporated to give a third batch of product. All three batches of product were triturated with MeOH, filtered off, washed with DE and dried in vacuo at 50⁰ to give the title compound (556mg) as a white solid (chars 195-9⁰; foams 210-211⁰).
T.l.c. DCM-MeOH (9:1), Rf 0.27
δ (DMSO) 1.6-2.1 (4H,m); 3.1-3.6 (4H,m); 4.65-4.9 (2H,s); 5.35-5.45 (1H,d); 7.2-7.9 (13H,m); 8.15-8.35 (1H,s); 9.3-9.5 (1H,s)

### Example 3

### N-[3-[[[(2,3-Dihydro-2-oxo-1-[2-oxo-2-(1-pyrrolidinyl)ethyl]-5-phenyl-1H-1,4-benzodiazepin-3-yl]amino]carbonyl]amino]phenyl]-1,1,1-trifluoromethanesulphonamide

A mixture of Intermediate 4 (565mg) and N-(3-aminophenyl)-1,1,1-trifluoromethanesulphonamide hydrochloride (450mg) in dry DCM (5ml) was treated with TEA (0.4ml) and the reaction mixture allowed to stir at room temperature overnight. The reaction mixture was diluted with DCM and then washed with dilute HCl, water, brine, dried (Na₂SO₄) and evaporated to give a beige foam. This was triturated with DCM/DE and the solid filtered to give the title compound (281 mg), m.p. 169-172⁰.
T.l.c. DCM-MeOH (9:1), Rf 0.5
δ (DMSO) 1.7-2.0 (4H,m); 3.2-3.6 (4H,m); 4.77 (2H,s); 5.33 (2H,d); 6.83 (1H,d); 7.2-7.75 (13H,m); 9.31 (1H,s); 11.8 (1H,br.s)

### Example 4

### 3-[[[[2,3-Dihydro-2-oxo-1-[2-oxo-2-(1-pyrrolidinyl)ethyl]-5-phenyl-1H-1,4-benzodiazepin-3-yl]amino]carbonyl]amino]-N-(methylsulphonyl)benzamide

A solution of Intermediate 4 (0.478g) in DCM (5ml) was treated with TEA (0.22ml) and Intermediate 6 (0.34g). The mixture was stirred at room temperature for 20h, DCM was added and the mixture washed with 2N HCl. The organic portion was dried and evaporated, the residue was triturated with DE and filtered to give the title compound (0.193g) as a white solid, m.p. 178-180⁰.
T.l.c. MeOH-DCM (5:95), Rf 0.30
δ (DMSO) 1.85 (4H,m); 3.35 (4H+H₂O,m); 3.40 (3H+H₂O,s); 4.80 (2H,s); 5.39 (1H,d); 7.25-7.80 (13H,m); 8.03 (1H,m); 9.40 (1H,s); 12.15 (1H,br.s)

### Example 5

### N-[[3-[[[[2,3-Dihydro-2-oxo-1-[2-oxo-2-(1-pyrrolidinyl)ethyl]-5-phenyl-1H-1,4-benzodiazepin-3-yl]amino]carbonyl]amino]phenyl]methyl]-1,1,1-trifluoromethanesulphonamide

A solution of Intermediate 4 (0.478g) in DCM (5ml) was treated with TEA (0.22ml) and Intermediate 8 (0.40g). The mixture was stirred at room temperature for 20h, DCM was added and the mixture was washed with 2N HCl. The organic portion was dried and evaporated, the residue was triturated with DE and filtered to give the title compound (0.152g) as a buff solid, m.p. 149⁰ (dec.).
T.l.c.MeOH-DCM (5:95), Rf 0.37
δ (DMSO) 1.85 (4H,m); 3.35 (4H+H₂O,m); 4.30 (2H,d); 4.77 (2H,s); 5.34 (1H,d); 6.94 (1H,m); 7.15-7.75 (13H,m); 9.20 (1H,s); 9.96 (1H,t)

### Example 6

### N-[2,3-Dihydro-2-oxo-1-[2-oxo-2-(1-pyrrolidinyl)ethyl]-5-phenyl-1H-1,4-benzodiazepi n-3-yl]-N'-[3-(2H-2-methyltetrazol-5-yl)phenyl]urea

A mixture of Intermediate 4 (460mg) and Intermediate 11 (310mg), in DCM (10ml) was treated with TEA (0.5ml), and the reaction mixture heated at reflux for 24h. The DCM was removed in vacuo, the residue suspended in dry THF (15ml), and heated at reflux for 3 days. The THF was removed in vacuo and the residue dissolved in DCM. The DCM solution was washed with dilute HCl solution, water, saturated brine, dried (Na₂SO₄) and concentrated to give a white solid. This was chromatographed eluting with DCM-MeOH; (9:1) to give the title compound as a white solid (131mg). m.p. 225-230°C (dec).
T.l.c.DCM-MeOH (9:1) Rf 0.27 δ (DMSO) 1.75-2.0 (4H,m); 3.25-3.6 (4H,m); 4.45 (3H,s); 4.8 (2H,s); 5.4 (1H,d);
7.1-7.8 (13H,m); 8.35 (1H,s);9.5 (1H,s).

### Example 7

### 3-[[[[2,3-Dihydro-2-oxo-1-[2-oxo-2-(1-pyrrolidinyl)ethyl]-5-phenyl-1H-1,4-benzodiazepin-3-yl]amino]carbonyl]amino]-N-(1H-tetrazol-5-yl)benzamide

A mixture of Intermediate 12 (0.380g) and carbonyldiimidazole (0.141g) in dry acetonitrile (30ml) was heated at 80° for 30 mins under nitrogen. The mixture was cooled and 5-aminotetrazole (0.074g) and TEA (0.24ml) were added and the mixture was heated at 80° for 20 hrs. The solvent was removed by evaporation and the residue chromatographed on silica, eluting with MeOH- acetic acid- DCM (3:1:96) to give the title compound (0.151g) as a white solid, mp 186-188°c
T.l.c. methanol-acetic acid-DCM (5:1:94) Rf 0.27
δ (DMSO) 1.8 (4H,m); 3.4(4H+N20,m) :4.8 (2H,s): 5.4 (1H,d); 7.2-7.8(13H,m); 8.0(1H,s); 9.3(1H,s); 12.0 (1H.brs.); 15.9 (1H,brs).

### Example 8

### N-[[3-[[[[2,3-Dihydro-2-oxo-1-[2-oxo-2-(1pyrrolidinyl)ethyl]-5-phenyl-1H-1,4-benzodiazepin-3-yl]amino]carbonyl]amino]phenyl]sulphonyl]acetamide

A mixture of intermediate 4 (0.435g), intermediate 14 (0.224g) and TEA (0.17ml) in dry THF (10ml) and dry DMF (10ml) was heated at 80° for 20hrs under nitrogen. The solvent was removed by evaporation and the residue was partitioned between 2N HCl and DCM, the organic portion was washed with 2N HCl, dried and evaporated. The residue was chromatographed eluting with methanol : DCM (3:97) to give the title compound (0.075g) as a white solid m.p. 193-195⁰.
T.l.c. MeOH-DCM (5:95) R.f 0.27
δ (DMSO) 1.8 (4H,m); 1.9 (3H,s); 3.4 (4H + H₂O; m); 4.8 (2H,m): 5.4. (1H,d); 7.2 - 7.8 (12H,m); 8.0 (1H,m); 9.4 (1H,s); 12.0 (1H,brs).

### Example 9

### N-[2,3-Dihydro-2-oxo-1-[2-oxo-2-(1-pyrrolidinyl)ethyl]-5-phenyl-1H-1,4-benzodiazepi n-3-yl]-N'-[3-(1H-tetrazol-5-ylmethyl)phenyl]urea

A mixture of Intermediate 4 (500mg) and Intermediate 16 (560mg) in DCM (10ml) was treated with triethylamine (0.4ml) and the reaction mixture heated at reflux for 24h. The reaction mixture was diluted with DCM and then washed with dilute HCl, water, saturated brine, dried (Na₂SO₄) and concentrated to give a beige solid. This was chromatographed eluting with DCM-MeOH; 9:1, to give the title compound as a white powder (203mg), m.p. 175-180°C (dec.).
T.l.c. DCM-MeOH (9:1), Rf 0.44
δ (CDCl₃) 1.45-1.7 (4H,m); 2.39 (1H,d); 2.45-2.6 (2H,m); 2.9-3.2 (2H,m); 3.6 (1H,d); 4.2 (1H,d); 5.2 (1H,d); 5.4 (1H,d); 6.45 (1H,s); 6.7 (1H,d); 6.95-7.65 (11H,m); 7.75 (1H,d); 8.34 (1H,d); 8.71 (1H,s)

### Example 10

### N-[2,3-Dihydro-2-oxo-1-[2-oxo-2-(1-pyrrolidinyl)ethyl]-5-phenyl-1H-1,4-benzodiazepin-3-yl]-N'-[3-(5-trifluoromethyl)-1H-1,2,4-triazol-3-yl]phenyl]urea

A mixture of the Intermediate 4 (500mg), Intermediate 19 (350mg) and TEA (0.2ml) in THF/DMF (1:1, 12ml) was heated at 80⁰C for 3 days. The solvents were removed in vacuo and the residue dissolved in DCM and washed with dilute HCl, dried and concentrated to give a brown gum. This was chromatographed (eluting with DCM-MeOH; 95:5) to give the title compound as a white solid (230mg). m.p. 203-207⁰C(dec).
T.l.c. MeOH-DCM (5:95) Rf 0.25.
δ (DMSO) 1.7-20(4H,m); 3.2-3.6 (4H,s); 4.8(2H,s) 5.4 (1H,s); 7.3-7.8(13H,m); 8.25(1H,s); 9.4(1H,s)

### Example 11

### 5-(2-Fluorophenyl)-2,3-dihydro-N-methyl-2-oxo-N-phenyl-3-[[[[3-(1H-tetrazol-5-yl)phenyl]amino]carbonyl]amino]-1H-1,4-benzodiazepine-1-acetamide

A solution of intermediate 22 (400mg) and CDI (156mg) in THF (25ml) was heated at reflux for 1½h. 3-(1H-tetrazol-5-yl)benzenamine hydrochloride (190mg) was added and heating at reflux was continued for 18h. The solvent was evaporated and the mixture dissolved in EA/water. The aqueous layer was acidified with 2N HCl and was extracted with EA. The combined organic extracts were washed with saturated brine, dried and evaporated to give a residue which was triturated with MeOH to give the title compound as a white powder (330mg), m.p. 210° dec.
T.l.c. DCM-MeOH (95:5), Rf 0.15
δ (DMSO) 3.25 (3H,s); 4.4 (2H,br.s); 5.3 (1H,d); 7.1-7.8 (17H,m); 8.1 (1H,s); 9.3 (1H,s)

### Example 12

### 5-(2-Fluorophenyl)-2,3-dihydro-N-methyl-2-oxo-N-phenyl-3-[[[[3-[(trifluoromethyl)sulphonylamino]phenyl]amino]carbonyl]amino]-1H-1,4-benzodiazepin e-1-acetamide

CDI (135mg) was added to a solution of Intermediate 22 (340mg) in dry THF (10ml) at 23° under nitrogen and the mixture was heated to reflux for 45min. N-(3-Aminophenyl)-1,1,1- trifluoromethanesulphonamide (195mg) in dry THF (3ml) was added and heating at reflux was continued for 72h. The cooled mixture was evaporated then redissolved with EA and phosphate buffer (pH6). The mixture was washed with saturated brine, dried and evaporated. The residue was chromatographed with hexane-THF to give the title compound as a white solid, m.p. 163-167°
T.l.c. hexane - THF (1:1) Rf 0.35
δ (DMSO) 3.2 (3H,s); 4.3-4.5.(2H,m);5.25-5.35(1H,d); 6.8(1H,d); 7.15-7.8(17H,m); 9.2(1H,s).

### Example 13a

### 5-(2-Fluorophenyl)-2,3-dihydro-N-methyl-2-oxo-N-phenyl-3-[[[[3-(1H-tetrazol-5-yl)phenyl]amino]carbonyl]amino]-1H-1,4-benzodiazepine-1-acetamide (isomer 1)

CDI (85mg) was added to a solution of the chiral amine Intermediate 23 (216mg) in dry THF (6ml) at 23° under nitrogen. After 45min, the 3-(1H-tetrazol-5-yl)benzenamine hydrochloride (123mg) was added and the mixture heated under reflux for 17h. The cooled reaction mixture was then partitioned between 1N HCl and EA. The combined organic extracts were washed with saturated brine and evaporated to dryness. The residual yellow foam was repeatedly taken into MeOH and a solid precipitated with DE. The combined solids were finally triturated with EA-DE and dried in vacuo to give the title compound (156mg) as a white solid, m.p. 206° dec.
T.l.c. DCM-MeOH (9:1), Rf 0.41
δ (DMSO) 3.2 (3H,s); 4.4 (2H,s); 5.35 (1H,d); 7.2-7.75 (17H,m); 8.15 (1H,s); 9.4 (1H,s)

### Example 13b

### 5-(2-Fluorophenyl)-2,3-dihydro-N-methyl-2-oxo-N-phenyl-3-[[[[3-(1H-tetrazol-5-yl)phenyl]amino]carbonyl]amino]-1H-1,4-benzodiazepine-1-acetamide (isomer 2)

CDI (85mg) was added to a solution of the chiral amine Intermediate 24 (213mg) in dry THF (6ml) at 23° under nitrogen. After 45min 3- (1H-tetrazol-5-yl)benzenamine hydrochloride (123mg) was added and the mixture heated under reflux for 17h. It was then cooled and partitioned between water and EA. The combined organic extracts were washed consecutively with 1N HCl and saturated brine, dried and evaporated. The residual foam was repeatedly taken into MeOH and solid precipitated with DE which was filtered off and dried in vacuo to give the title compound (228mg) as a cream solid, m.p. 191-5° dec.
T.l.c. DCM-MeOH (9:1), Rf 0.42
δ (DMSO) 3.1 (3H,s); 4.4 (2H,s); 5.35 (1H,d); 7.2-7.75 (17H,m); 8.15 (1H,s); 9.25 (1H,s)

### Example 14

### N-[[3-[[[[5-(2-Fluorophenyl)-2,3-dihydro-2-oxo-1-[2-oxo-2-(1-pyrrolidinyl)ethyl]-1H-1,4-benzodiazepin-3-yl]amino]carbonyl]amino]phenyl]-1,1,1-trifluoromethanesulphonamide

CDI (135mg) was added to a solution of Intermediate 29 (310mg) in dry THF (10ml) at 23° under nitrogen and the mixture was heated to reflux for 45 mins. N-(3-Aminophenyl)-1,1,1- trifluoromethanesulphonamide (195mg) in dry THF (3ml) was added and heating at reflux continued for 72h. The cooled mixture was evaporated then redissolved with EA and 2N HCl. The mixture was extracted with EA and the combined extracts were washed with saturated brine, dried and evaporated. Trituration with DE gave the title compound (60mg) as a buff solid, m.p. 225° dec.
T.l.c. THF-hexane (1:1), Rf 0.13
δ (DMSO) 1.7-2.0 (4H, m): 3.35 - 3.6 (4H,m); 4.6 - 4.9 (2H,m); 5.35 (1H,d); 6.7 - 7.8 (13H,m); 9.15 (1H,s)

### Example 15

### N-[5-(2-Fluorophenyl)-2,3-dihydro-2-oxo-1-[2-oxo-2-(1-pyrrolidinyl)ethyl]-1H-1,4 benzodiaepin-3-yl]-N'-[3-(1H-tetrazol-5- yl)phenyl]urea

A solution of Intermediate 29 (0.5g) and CDI(0.22g) in THF (10ml) was heated to reflux under nitrogen for 1½h. 3-(1H-tetrazol-5- yl)benzenamine hydrochloride (0.26g) was added and heating was continued for 18h. The reaction mixture was evaporated then redissolved with EA and 0.6N HCl. The aqueous layer was extracted with EA and DCM. The combined organic extracts were dried and evaporated to give a brown foam which after trituration with MeOH gave the title compound as a white powder (0.26g), m.p. 214° decomposed.
T.l.c. DCM-MeOH (95:5), Rf 0.18
δ (DMSO) 1.7-2.1 (4H,m); 2.7-4.3 (4H+H₂O;m); 4.5-4.9 (2H,m); 5.4 (1H ,d); 7.1-7.8 (12H,m); 8.15 (1H,s); 9.3 (1H,s)

### Example 16

### 3-[[[[2,3-Dihydro-N-methyl-2-oxo-N,5-diphenyl-1H-1,4-benzodiazpin-3-yl] amino]carbonyl]aminol-N-(1H-tetrazol-5-yl)benzamide

A mixture of Intermediate 31 (480mg) and CDI (152mg) in dry acetonitrile (30ml) was heated at reflux for 30mins. The mixture was cooled to room temperature and 5-aminotetrazole (80mg) and triethylamine (0.26ml) were added. The resulting mixture was heated at reflux for 20hrs. The solvent was removed by evaporation and the residue was columned eluting with 3% MeOH-1% AcOH-DCM to give the title compound (430mg) as a white solid mp 184-186°
T.l.c. (5% MeOH, 1% AcOH, DCM) Rf 0.22
ir 700, 1240, 1325, 1380, 1594, 1668cm⁻¹

### Example 17

### N-[[3-[[[[2,3-Dihydro-N-methyl-2-oxo-N,5-diphenyl-1H-1,4-benzodiazepin-3-yl] amino]carbonyl]amino]phenyl]sulphonyl]acetamide

A solution of Intermediate 30 (500mg) in dry DMF (5ml) was added to a mixture of Intermediate 14 (260mg) and triethylamine (0.17ml) in dry THF (10ml). The resulting mixture was heated at 70° for 64hrs. The mixture was poured into 2N HCl (30ml) extracted with ethylacetate (5x15ml). The combined extracts were dried and evaporated to give the title compound (163mg) as a white solid. m.p. 168-169°
T.l.c. (MeOH-DCM 5:95) Rf 0.43
ir 1337, 1547, 1596, 1668cm⁻¹

### Example 18

### 3-[[[[2,3-Dihydro-N-methyl-2-oxo-N,5-diphenyl-1H-1,4-benzodiazepin-3- yl]amino] carbonyl]amino]-N-(methylsulphonyl)benzamide

A solution of Intermediate 30 (500mg) in dry THF (5ml) was added to a stirring mixture of Intermediate 6 (260mg) and triethylamine (170µl) in THF (10ml). The resulting mixture was heated at 70° for 64hrs. The solvent was removed by evaporation and the residue was columned eluting with MeOH-DCM, (5:95) to give the title compound (390mg) as a white solid m.p. 224-226°
T.l.c. MeOH-AcOH-DCM, 5:1:94) Rf 0.58
ir 1153, 1216, 1335, 1650, 1690cm⁻¹

### Example 19

### 2,3-Dihydro-N-ethyl-2-oxo-N,5-diphenyl-3-[[[[3-(1H-tetrazol-5- yl)phenyl]amino] carbonyl]amino]-1H-1,4-benzodiazepine-1-acetamide

CDI (72mg) was added to a solution of Intermediate 34 (183mg) in THF (8ml) at 23° under nitrogen. After 1h 40min more CDI (20mg) was added and stirring continued for 45min as the solution was heated to reflux. 3-(1H-Tetrazol-5-yl)benzenamine (113mg) was added and the resulting suspension stirred under reflux for 15h. The white solid was filtered from the cooled mixture, triturated with MeOH, re-filtered, washed with DE and dried in vacuo at 50° to give the title compound (155mg) as a beige solid, m.p. 224-5° dec
T.l.c. (9:1 DCM-MeOH) Rf 0.44
δ (DMSO) 1.05 (3H,t); 3.3-4.0(2H, masked by water in solvent) 4.3 (2H,m); 5.3 (1H,d); 7.2(1H,s); 7.3-7.8 (11H, m); 7.95 (1H,s); 8.05 (1H,s);9.28(1H,s)

### Example 20

### N-[2,3-Dihydro-1-(3-methylbutyl)-2-oxo-5-phenyl-1H-1,4-benzodiazepin-3-yl]-N'-[3-(1H-tetrazol-5-yl)phenyl]urea

A solution of Intermediate 35 (150mg) in dry THF (5ml) was treated sequentially with TEA (72µl) and 3-(1H-tetrazol-5- yl)benzenamine (102mg). The suspension was then heated under reflux for 23h, cooled and poured into 2N HCl (30ml) then extracted with EA (2x40ml). A precipitate formed in the combined organic extracts which was filtered off. The filtrate was evaporated and the residue triturated with DE, filtered off, combined with the first filter- cake and dried in vacuo to give the title compound (147mg) as a beige solid, m.p. 186-9⁰ dec.
T.l.c. DCM-MeOH (9:1), Rf 0.5
δ (DMSO) 0.6-0.8(6H,dd); 1.2-1.3(3H,m); 3.5-4.5(2H,2xm); 5.2- 5.35(1H,d); 7.3-7.9(13H,m); 8.2-8.25(1H,s); 9.4-9.5(1H,s).

### Example 21

### N-[1-(3,3-Dimethyl-2-oxobutyl)-2,3-dihydro-2-oxo-5-phenyl-1H-1,4-benzodiazepin-3 -yl]-N'-[3-(1H-tetrazol-5-yl)phenyl]urea

CDI (106mg) was added to a solution of Intermediate 38 (229mg) in dry THF (3ml) at 23⁰ under nitrogen. After 40min the orange solution was treated with TEA (0.1ml) and 3-(1H-tetrazol-5- yl)benzenamine (142mg). Stirring was continued at 23⁰ for 5h whereupon more solvent (3ml) was added and the orange suspension heated under reflux for 1.5h. Further charges of TEA (40µl) and 3-(1H-tetrzole-5-yl)benzenamine (56mg) were added and heating continued under reflux for 16h whereupon the cooled orange mixture was partitioned between 2N HC1 and EA. The combined organic phases were washed with saturated brine, dried and evaporated. The residue was triturated with EA-DE and dried in vacuo to give the title compound (220mg) as an off- white solid, m.p. 193⁰ dec.
T.l.c. (9:1 DCM-MeOH), Rf 0.45
δ (DMSO) 1.1-1.15(9H,s); 4.9-5.1(2H,m); 5.3-5.35(1H,d); 7.25- 7.8(13H,m); 8.1-8.15(1H,s); 9.2-9.3(1H,s).

### Example 22

### N-[1-(2-Phenyl-2-oxoethyl)-2,3-dihydro-2-oxo-5-phenyl-1H,4-benzodiazepin-3-yl]-N^{¹}-[3-(1H-tetrazol-5-yl)phenyl]urea

NaH (80% in oil; 21mg) was added to a cloudy solution of Intermediate 40 (100mg) in dry DMF (2ml) at 0° under nitrogen. After 25min phenacyl bromide (55mg) was added and the yellow solution stirred at 0° for 30 min whereupon it was poured carefully into phosphate buffer solution (pH6.5; 20ml) and extracted with EA (2x20ml). The combined extracts were washed with saturated brine and evaporated. The residue was triturated with MeOH filtered off, washed with DE and dried in vacuo to give a white solid. This solid was stirred with water (6ml) for 1h at 23° and the suspension filtered. The solid was washed with MeOH then dried in vacuo at 70° for 2h to give the title compound (38mg) as a white solid. m.p. 195° dec, foams.
T.l.c. (9:1 DCM-MeOH) Rf 0.5
δ (DMSO) 5.45(1H,d); 5.6(2H,dd); 7.25-8.10 (14H,m), 9.3(1H,s)

### Example 23

### N-[1-(2-Cyclopentyl-2-oxoethyl)-2,3-dihydro-2-oxo-5-phenyl-1H-1,4-benzodiazepin-3-yl]-N'-[3-(1H-tetrazol-5-yl)phenyl]urea

CDI (133mg) was added to a solution of Intermediate 43 (269mg) in dry THF (10ml) at 23° and the mixture was heated to reflux. After 30min 3(1H-tetrazol-5-yl)benzenamine hydrochloride (176mg) was added and heating at reflux was continued for 18h. The reaction mixture was poured into phosphate buffer (pH6; 100ml) and extracted with EA. The combined extracts were washed with brine, dried (MgSO₄) and evaporated. The residue was triturated with MeOH to give the title compound (256mg) as a solid, m.p. 172-175°.
T.l.c.5% MeOH in DCM, Rf 0.10
δ (DMSO) 1.4-1.9 (8H,m); 3.0-3.1 (1H,m); 4.9-5.0 (2H,br s); 5.3-5.4 (1H,d); 7.1-7.8 (13H,m); 8.2 (1H,s); 9.35 (1H,s)

### Example 24

### N-[3-[[[[1-(2-cyclopentyl-2-oxoethyl)-2,3-dihydro-2-oxo-5-phenyl-1H-1,4-benzodiazepin-3-yl]amino]carbonyl]amino]phenyl]-1,1,1-trifluoromethanesulphonamide

Dry THF (3ml) was added to a stirred mixture of Intermediate 43 (135mg) and CDI (61mg) at 23° under nitrogen and the mixture was left to stir for 30 minutes. Aniline triflamide (103mg) was added and the mixture was heated to reflux for 66 hours. After cooling the mixture was poured into pH6.5 phosphate buffer solution (100ml) and was extracted with EA (100mlx2). The combined organic extracts were washed with saturated brine, dried and evaporated in vacuo. The crude product was triturated with MeOH then purified by flash chromatography on acid-washed silica (Merck 9385; 5g) using DCM - MeOH (2% 5%) as eluent to give product as a cream white powder (82mg;) m.p. 175°dec.
T.l.c. (5% MeOH-DCM) Rf 0.29
δ (DMSO) 1.7-2.0(8H,m); 3.5(1H,m); 5.0(2H,s),5.4(1H,d), 7.3-7.9(13H,m),8.55(1H,s), 11.9(1H,brs).

### CCK-B Antagonist Activity

The CCK-B antagonist activity of the compounds of the invention was determined using the guinea pig isolated ileum longitudinal muscle-myenteric plexus preparation. The compounds were tested using the procedure of Patel M and Spraggs C.F. Br. J Pharmacol (1992) 106, 275-282 and the pKb value for each compound was determined using the conventional Goddam analysis. The results obtained with representative compounds of the invention were as follows:

| Compound of Ex No. | pKb |
|---|---|
| 1 | 11.6 |
| 2 | 10.3 |
| 5 | 9.5 |
| 6 | 10.5 |
| 7 | 10.1 |
| 8 | 10.1 |
| 10 | 9.8 |
| 11 | >10 |
| 17 | 11.7 |
| 18 | 11.4 |
| 19 | 10.9 |
| 23 | 11 |

The compounds of the invention are essentially non toxic at therapeutically useful doses. Thus for example no untoward effects were observed when the compounds were given (iv) to Heidenhain pouch dogs at doses which inhibited pentagastrin induced gastric acid secretion.

### Pharmacy Examples

### Tablets

| | | |
|---|---|---|
| a. | Active ingredient | 50mg |
| | Lactose anhydrous USP | 163mg |
| | Microcrystalline Cellulose NF | 69mg |
| | Pregelatinised starch Ph.Eur. | 15mg |
| | Magnesium stearate USP | 3mg |
| | Compression weight | 300mg |

The active ingredient, microcrystalline cellulose, lactose and pregelatinised starch are sieved through a 500 micorn sieve and blended in a suitable mixer. The magnesium stearate is sieved through a 250 micron sieve and blended with the active blend. The blend is compressed into tablets using a suitable punches.

| | | |
|---|---|---|
| b. | Active ingredient | 50mg |
| | Lactose monohydrate USP | 120mg |
| | Pregelatinised starch Ph.Eur. | 20mg |
| | Crospovidone NF | 8mg |
| | Magnesium stearate USP | 2mg |
| | Compression weight | 200mg |

The active ingredient, latose and pregelatinised starch are blended together and granulated with water. The wet mass is dried and milled. The magnesium sterate and Crospovidone are screened through a 250 micron sieve and blended with the granule. The resultant blend is compressed using suitable tablet punches.

### Capsules

| | | |
|---|---|---|
| a. | Active ingredient | 50mg |
| | Pregelatinised Starch Ph.Eur. | 148mg |
| | Magnesium stearate USP | 2mg |
| | Fill weight | 200mg |

The active ingredient and pregelatinised starch are screened through a 500 micron mesh sieve, blended together and lubricated with magnesium stearate (meshed through a 250 micron sieve). The blend is filled into hard gelatin capsules of a suitable size.

| | | |
|---|---|---|
| b. | Active ingredient | 50mg |
| | Lactorse monohydrate USP | 223mg |
| | Povidone USP | 12mg |
| | Crospovidone NF | 12mg |
| | Magnesium stearate | 3mg |
| | Fill weight | 300mg |

The active ingredient and lactose are blended together and granulated with a solution of Povidone. The wet mass is dried and milled. The magnesium stearate and Crospovidone are screened through a 250 micron sieve and blended with the granule. The resultant blend is filled into hard gelatin capsules of a suitable size.

A preferred active ingredient for use in the pharmacy examples is the compound of Example 18.

## Claims

1. Compounds of general formula (I) 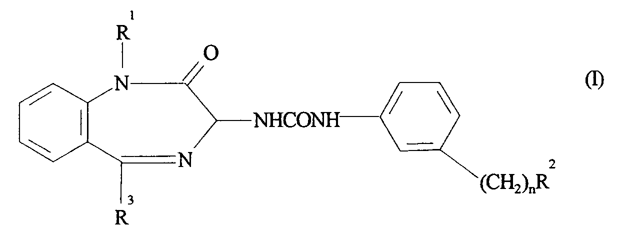 wherein
R¹ represents a group selected from CH₂CONR⁴R⁵, XYR⁶, phenyl, C₃₋₇-cycloalkyl or C₁₋₆alkyl, optionally substituted by a hydroxy, phenyl, C₁₋₆alkoxycarbonyl, C₃₋₇cycloalkyl or adamantyl group;
R² represents a group selected from NR⁷SO₂CF₃, SO₂NR⁷COR⁸, CONR⁷SO₂R⁸,
or a tetrazole, carboxamidotetrazole, or 3-trifluoromethyl-1,2,4- triazole group in which the tetrazole or triazole moiety may be substituted on one of the nitrogen atoms by a C₁₋₄alkyl group;
R³ is phenyl optionally substituted by one or two halogen atoms;
R⁴ and R⁵ which may be the same or different each independently represent a hydrogen atom, or a phenyl or C₁₋₄alkyl group or NR⁴R⁵ represents a saturated 5- to 7- membered nitrogen containing heterocyclic ring, optionally substituted by 1 or 2 methyl groups;
R⁶ represents a group selected from C₁₋₆alkyl, optionally substituted phenyl, C₃₋₇cycloalkyl or adamantyl;
R⁷ represents hydrogen or a C₁₋₄alkyl group;
R⁸ represents a C₁₋₄alkyl group,
X is a C₁₋₃ straight or branched alkylene chain;
Y represents a group selected from -C=O, C(OR⁹)₂ or C(SR⁹)₂ wherein R⁹ is C₁₋₃alkyl or the two R⁹ groups together form a C₂₋₄alkylene chain;
n is zero or 1; and pharmaceutically acceptable salts and solvates thereof.

2. Compounds as claimed in Claim 1 wherein R¹ represents the group XYR⁶

3. Compounds as claimed in Claim 1 wherein R¹ represents the group CH₂CONR⁴R⁵

4. Compounds as claimed in Claims 1 and 3 wherein R⁴ is phenyl and R⁵ represents C₁-4 alkyl.

5. Compounds as claimed in Claim 4 wherein R⁵ is methyl

6. Compounds as claimed in any one of Claims 1 to 5 wherein R³ is phenyl or_ o-fluorophenyl

7. Compounds as claimed in any of Claims 1 to 6 wherein R² is a group selected from -NHSO₂CF₃ - SO₂NHCOCH₃ CONHSO₂CH₃

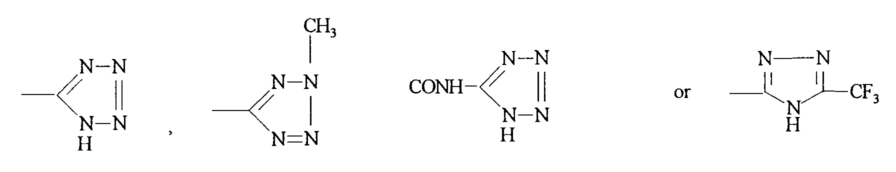

8. Compounds as claimed in any of claims 1 to 7 wherein R2 is a group selected from CONHSO₂CH₃ or 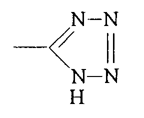 and n is zero

9. 3-[[[[2,3-Dihydro-N-methyl-2-oxo-N,5-diphenyl-1H-1,4-benodiazepin-3- yl]amino] carbonyl]amino]-N-(methylsulphonyl)benzamide and enantiomers thereof;

10. 2,3-Dihydro-N-methyl-2-oxo-N,5-diphenyl-3-[[[[3-(1H-tetrazol-5-yl)phenyl)amino] carbonyl]amino]-1H-1,4-benzodiazepine-1-acetamide;
5-(2-Fluorophenyl)-2,3-dihydro-N-methyl-2-oxo-N-phenyl-3-[[[[3-(1H-tetrazol-5-yl)phenyl]amino]carbonyl]amino]-1H-1,4-benzodiazepine-1-acetamide;
N-[2,3-Dihydro-2-oxo-1-[2-oxo-2-(1-pyrrolidinyl)ethyl]-5-phenyl-1H-1,4-benzodiazepin-3-yl]-N'-[3-(1H-tetrazol-5-yl)phenyl]urea; and enantiomers thereof

11. Compounds as claimed in any of Claims 1 to 10 for use in therapy

12. The use of a compound as claimed in any one of Claims 1 to 10 in the manufacture of a medicament for the treatment of conditions where a modification of the effects of gastrin and or CCK is of therapeutic benefit.

13. Pharmaceutical compositions comprising a compound as claimed in any of Claims 1 to 10 in admixture with one or more physiologically acceptable carrier or excipients.

14. A method of treatment of a mammal including man, for conditions where modification of the effects of gastrin and or CCK is of therapeutic benefit comprising administration of an effective amount of a compound as claimed in any of claims 1 to 10.

15. A process for the preparation of compounds as defined in Claim 1 which comprises
a) reacting a compound of formula (II) with an aniline of formula (III) or an acid addition salt thereof in which R² has the meanings as defined in formula (I) or is a group convertible thereto
b) acylating an amine of formula (IV) by reaction with an isocyanate of formula (V) or a carbamoyl chloride of formula (VI) 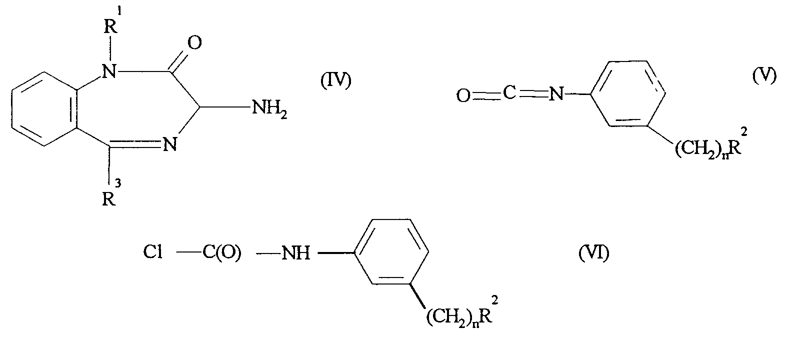
c) alkylating a compound of formula (I) wherein R¹ is hydrogen by reaction with the appropriate halide R¹ hal wherein R¹ has the meaning as defined in formula (I) and hal represents halogen;
and thereafler if necessary or desired subjecting the resulting compounds to one or more of the following steps:
(i) conversion of one compound of formula (I) into other compounds of formula I
(ii) formation of a pharmaceutically acceptable salt thereof.
